(19) Europäisches Patentamt
European Patent Office
Office européen des brevets

(11) **EP 4 559 382 A1**

(12) **EUROPEAN PATENT APPLICATION**

(43) Date of publication:
**28.05.2025 Bulletin 2025/22**

(21) Application number: **24213681.0**

(22) Date of filing: **18.11.2024**

(51) International Patent Classification (IPC):
**A61B 5/00** (2006.01)   **A61B 5/08** (2006.01)
**A61B 5/053** (2021.01)   **A61B 5/318** (2021.01)

(52) Cooperative Patent Classification (CPC):
**A61B 5/7203; A61B 5/318; A61B 5/746;**
A61B 5/053; A61B 5/0816

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **21.11.2023 US 202363601325 P**

(71) Applicant: **Draeger Medical Systems, Inc.**
**Andover, MA 01810 (US)**

(72) Inventors:
• **MURRAY, William Joseph**
**Wakefield, 01880 (US)**
• **ZUZARTE, Ian Jeromino**
**Chelmsford, 01824 (US)**

(74) Representative: **Haseltine Lake Kempner LLP**
**One Portwall Square**
**Portwall Lane**
**Bristol BS1 6BH (GB)**

(54) **NON-LINEAR HEART RATE ADAPTIVE CARDIAC ARTIFACT FILTER FOR AN IMPEDANCE RESPIRATION SIGNAL**

(57)    The present disclosure provides a non-linear, heart rate adaptive, cardiac artifact filter designed to reduce cardiac artifacts in a patient's impedance respiration signal. The patient's impedance respiration signal may be acquired using electrocardiogram ("ECG") sensors and may be impacted by the patient's cardiac activity. This impact occurs at a single dynamically changing frequency determined from heart rate measurement. The adaptive cardiac artifact filter can be used to filter unwanted cardiac artifacts from the impedance respiration signal to provide a more accurate representation of the patient's cardiac activity. A filtered impedance respiration signal can then be displayed or analyzed to further the monitoring and treatment of the patient. The heart rate adaptive, cardiac artifact filter may be implemented in a physiological monitoring device that may be, in turn, a part of a system.

*FIG. 1*

EP 4 559 382 A1

**Description**

TECHNICAL FIELD

The present disclosure relates generally to the field of medical monitoring of physiological parameters in a patient and, more particularly, to monitoring a patient's condition using an electrocardiogram ("ECG") and impedance respiration.

BACKGROUND

This section of this document introduces information about and/or from the art that may provide context for or be related to the subject matter described herein and/or claimed below. It provides background information to facilitate a better understanding of the various aspects of the present invention. This is a discussion of "related" art. That such art is related in no way implies that it is also "prior" art. The related art may or may not be prior art. The discussion in this section of this document is to be read in this light, and not as admissions of prior art.

Conventional, modern medical practice frequently includes measurement and/or monitoring of a patient's physiological condition. Commonly known physiological parameters that are measured and/or monitored might include, for example and without limitation, blood pressure, body temperature, vision acuity, quantities of various substances in the blood, etc. Because of the value imparted by this information, the art of medical monitoring has developed an array of instruments and procedures for acquiring this kind of information.

One well-known measurement/monitoring technique is an "electrocardiogram" ("ECG"). Electrocardiograms are commonly used to monitor patients' heart conditions as well as to detect or predict cardiac events and conditions. In clinical settings, ECG signals representative of a patient's condition are captured in waveforms and analyzed by physiological monitoring devices. However, ECG sensors are also useful for acquiring other kinds of medical information besides cardiac events and conditions.

An ECG graphs voltage acquired from a person's body over time. The voltages represent electrical activity of the heart that cause the heart to beat. To acquire the voltages, sensors, or "electrodes", are placed at selected points on the person's body. The number and location of the electrodes are fairly standardized but may vary depending on the type of medical information that is of interest. For example, in one common nomenclature, the sensors are electrically connected to the physiological monitoring device by cables. In a clinical context, the sensors and cables together are sometimes referred to, or are colloquially known as, "leads". However, herein, they shall be more generally referred to as "sensor and cable assemblies".

SUMMARY

The present disclosure provides a non-linear, heart rate adaptive, cardiac artifact filter designed to reduce cardiac artifacts in a patient's impedance respiration signal. The lungs and heart expand into the same body cavity when a patient breathes and when the patient's heart beats. In some instances, signal artifact related to cardiac activity may be detected as part of the respiration signal. When it does happen, this interference may leave what is known as a "cardiac artifact" in the patient's impedance respiration signal. This cardiac artifact is undesirable.

The patient's impedance respiration signal may be acquired using, for example, ECG sensors. The patient's impedance respiration signal may be impacted by the patient's cardiac activity as described above. This impact occurs at a single dynamically changing frequency determined from heart rate measurement between, for example, 15 to 300 bpm ("beats per minute'), or, 0.25-5 Hz, although these numbers may vary in a given case. The adaptive cardiac filter can then be used to filter unwanted cardiac artifacts from the impedance respiration signal to provide a more accurate representation of the patient's respiratory activity. The filtered impedance respiration signal can then be displayed or analyzed to further the monitoring and treatment of the patient.

In one aspect, a method for non-linear, heart rate adaptive, cardiac artifact filtering an impedance respiration signal comprises: determining a varying heart rate of a monitored patient from an acquired electrocardiogram ("ECG") signal; acquiring an impedance respiration signal and removing a frequency component from the impedance respiration signal, the frequency component corresponding to the varying heart rate, to smooth the impedance respiration signal and filter out at least one cardiac artifact.

In yet another aspect, a method for monitoring a patient's physical condition comprises: acquiring an impedance respiration signal associated with the patient's cardiac activity; and applying a non-linear, heart rate adaptive, cardiac artifact filter to obtain a filtered impedance respiration signal.

In still another aspect, a physiological monitoring device comprises: a processor-based resource; and a memory encoded with instructions that, when executed by the processor-based resource, performs the method of the first aspect.

In another aspect, a system for physiologically monitoring a patient comprises: a plurality of electrocardiogram ("ECG") sensors; and a physiological monitoring device communicating with the plurality of ECG system and performing the method of the third aspect.

In yet another aspect, a method for non-linear, heart rate adaptive, cardiac artifact filtering is substantially as shown and described herein.

In still another aspect, a non-linear, heart rate adaptive, cardiac artifact filter is substantially as shown and described herein.

In another aspect, a method for monitoring a patient's physical condition is substantially as shown and described herein.

In still another aspect, a physiological monitoring device is substantially as shown and described herein.

In yet another aspect, a system for physiologically monitoring a patient is substantially as shown and described herein.

The above presents a simplified summary of the invention as claimed below in order to provide a basic understanding of some aspects of the invention. This summary is not an exhaustive overview of the invention. It is not intended to identify key or critical elements of the invention or to delineate the scope of the invention. Its sole purpose is to present some concepts in a simplified form as a prelude to the more detailed description that is discussed later.

BRIEF DESCRIPTION OF THE DRAWINGS

In the drawings, like reference numbers generally indicate identical, functionally similar, and/or structurally similar elements.

FIG. 1 illustrates a patient monitoring system according to one or more examples.

FIG. 2 is a schematic diagram of an example of a physiological monitoring device capable of executing a customizable physiological measurement process for measuring physiological parameters according to an embodiment of the present disclosure.

FIG. 3 is a schematic diagram of an example of a patient monitoring system including a computing system and the physiological monitoring system of FIG. 1 according to an embodiment of the present disclosure.

FIG. 4 presents a patient model illustrating these measurement and acquisition of the impedance respiration signals in the embodiments disclosed herein.

FIG. 5 illustrates a method for use in quantifying a physiological parameter of a monitored patient in accordance with one or more embodiments.

FIG. 6 schematically depicts one particular implementation of a physiological monitoring device.

FIG. 7 illustrates method for non-linear, heart rate adaptive, cardiac artifact filtering an impedance respiration signal in accordance with various embodiments disclosed herein.

FIG. 8 shows the filtering effect of the method of FIG. 7 when applied to real patient data.

FIG. 9 illustrates one particular embodiment for removing the frequency component from the impedance respiration signal.

While the disclosed subject matter is susceptible to various modifications and alternative forms, the drawings illustrate specific implementations described in detail by way of example. It should be understood, however, that the description herein of specific examples is not intended to limit that which is claimed to the particular forms disclosed, but on the contrary, the intention is to cover all modifications, equivalents, and alternatives falling within the spirit and scope of the appended claims.

DETAILED DESCRIPTION

Illustrative examples of the subject matter claimed below are disclosed. In the interest of clarity, not all features of an actual implementation are described for every example in this specification. It will be appreciated that in the development of any such actual implementation, numerous implementation-specific decisions may be made to achieve the developers' specific goals, such as compliance with system-related and business-related constraints, which will vary from one implementation to another. Moreover, it will be appreciated that such a development effort, even if complex and time-consuming, would be a routine undertaking for those of ordinary skill in the art having the benefit of this disclosure.

The physiological monitoring system disclosed herein is used, in accordance with the present disclosure, to measure "impedance respiration" ("IR"). Impedance respiration is an indirect method for measuring a patient's respiration rate ("RR"), which is a useful parameter in assessing a patient's health. Typical ranges for respiration rate are between 18 bpm and 60 bpm for neonates and 12 - 20 bpm for adults. Generally, respiration rate values outside of these ranges imply the patient may be in critical condition. Low respiration rate is called bradypnea while high respiration rate is called tachypnea. Impedance respiration is mainly used to monitor post-surgery patients who are under anesthesia and neonates. It is particularly useful for premature neonates, who are prone to apnea events (lack of breathing).

The technique utilizes the fact that the thoracic impedance of a person varies with time as a function of their breathing cycle. When inhaling, the air entering the lungs causes a small increase to thoracic impedance, while exhaling causes a small decrease. The peak-to-peak change in impedance due to breathing is usually between $0.3\,\Omega$ to $3\,\Omega$ for most patients. However, there is also a constant component to the thoracic impedance due to the air remaining always in the lungs, and the impedance of the bones, blood, etc. of the human body. This constant, or DC component, is in the order of $500\,\Omega$ to $2\,k\Omega$ for most patients.

Although the ECG technology and the measurement of impedance respiration is well established, challenges remain. Ideally, the impedance respiration signal is a sinusoidal signal whose frequency is the respiration rate. However, in practice analyzing the impedance respiration signal and extracting an accurate respiration rate from it is not always trivial.

For example, one challenge is the presence of cardiac artifacts in the impedance respiration signal. The pumping of the blood due to the function of the heart creates small but measurable changes in thoracic impedance. These so-called cardiac artifacts are of amplitude usually negligible compared to the impedance variations caused by breathing. However, in cases of shallow breathing these cardiac artifacts can be erroneously interpreted as breathing and cause false tachypnea alarms, often with a respiration rate close to the heart rate. The presence of cardiac artifacts can be particularly strong in neonates.

A second challenge is what are called motion or movement artifacts. Patient motion causes the skin-electrode impedance to vary with time, and such impedance variations can be erroneously interpreted as changes to impedance due to breathing, thus leading to false respiration readings and inaccurate respiration rates.

Another challenge is presented by apneas missed by caregivers due to noise or artifacts. Apnea is a life-threatening condition defined as a long pause in breathing and the respiration monitoring technique should sound an alarm to these events. As per the default settings an apnea alarm is sounded when there are no breaths detected for 15 seconds. Occasionally, noise or cardiac/motion artifacts can cause a breath to be detected during a real apneic event. This can cause the apnea alarm event to not be called and lead to a critical condition going undetected.

Yet another challenge is the detection of false apneas due to low impedance sensitivity. Missed apneic alarm can have fatal consequences. However, frequent false alarms can have similar consequences. High number of false alarms has led to alarm fatigue, a sensory overload when clinicians are exposed to an excessive number of alarms, resulting in desensitization to alarms and missed alarms. Patient deaths have been attributed to alarm fatigue. False apneic alarms can occur due to shallow breathing especially common in neonates. Shallow breathing could cause rapid transitions from normal impedance respiration to low impedance levels. Breaths during such low impedance should be accurately detected to avoid false alarms.

Those in the art having the benefit of this disclosure will appreciate that there may still be other challenges in the measurement of impedance respiration through ECG analysis. The implications of these and other challenges, as set forth above, can be quite serious. Accordingly, the presently disclosed technique is directed to resolving, or at least mitigating, these challenges.

The technique disclosed herein is disclosed in the context of one or more embodiments in which the impedance respiration is obtained in order to determine a patient's respiration rate. However, the technique is not limited to the ascertainment of impedance respiration. It is to be understood that the technique can be applied to quantify any patient physiological measurement interfered with by a different and separable signal source. Those in the art having the benefit of this disclosure will be able to readily adapt the disclosed technique to physiological parameters other than impedance respiration.

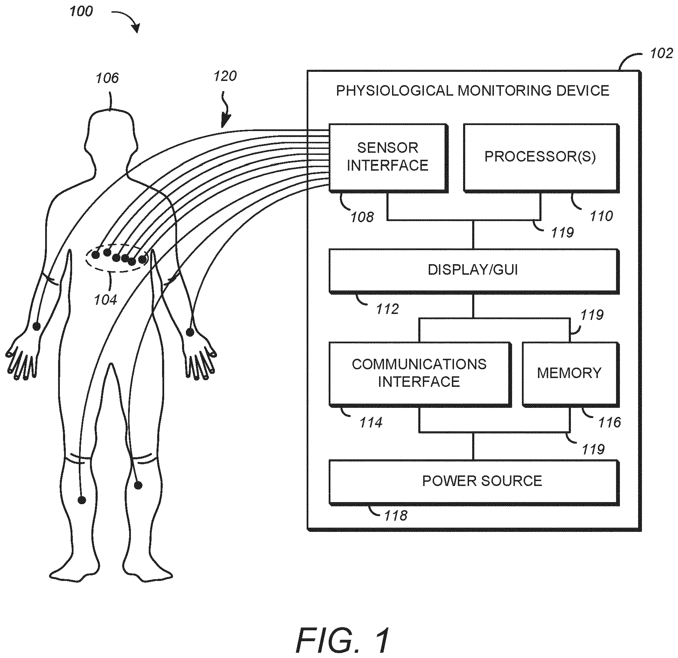

Turning now to the drawings, FIG. 1 illustrates a physiological monitoring system 100 according to one or more examples. As shown in FIG. 1, the system 100 includes a physiological monitoring device 102 capable of receiving physiological data from various sensors 104 connected to a patient 106 when deployed. In this example, the plurality of sensors 104 comprise electrocardiogram ("ECG") electrodes affixed to the skin of patient 106.

In general, it is contemplated by the present disclosure that physiological monitoring device 102 includes electronic components and/or electronic computing devices operable to receive, transmit, process, store, and/or manage patient data and information associated performing the functions of the system as described herein, which encompasses any suitable processing device adapted to perform computing tasks consistent with the execution of computer-readable instructions stored in a memory or a computer-readable recording medium.

Further, any, all, or some of the computing devices in physiological monitoring device 102 may be adapted to execute any operating system, including Linux®, UNIX®, Windows Server®, *etc.*, as well as virtual machines adapted to virtualize execution of a particular operating system, including customized and proprietary operating systems. Physiological monitoring device 102 may be further equipped with components to facilitate communication with other computing devices over one or more network connections, which may include connections to local and wide area networks, wireless and wired networks, public and private networks, and any other communication network enabling communication in the system.

As shown in FIG. 1, physiological monitoring device 102 may be, for example, a patient monitor implemented to monitor various physiological parameters of patient 106 via sensors 104. Physiological monitoring device 102 may include a sensor interface 108, one or more processors 110, a display/graphical user interface ("GUI") 112, a communications interface 114, a memory 116, and a power source (or power connection) 118, all communicating over an internal bus 119. The sensor interface 108 may be implemented in hardware or combination of hardware and software and is used to connect via wired and/or wireless connections to the sensors 104 for gathering physiological data from the patient 106.

As noted, the sensors 104 in the present example are ECG electrodes affixed to the skin of the patient 106. A plurality of sensor and cable assemblies 120, comprising a plurality of conductive cables, are provided for coupling the sensors 104 to the sensor interface 108. In one or more examples, the sensor and cable assemblies 120 comprise a plurality of ECG cables.

The data signals from the sensors 104 may include, for example, sensor data related to an ECG. The one or more processors 110 may be used for controlling the general operations of the physiological monitoring device 102, as well as processing sensor data received by sensor interface 108. The one or more processors 110 may be any suitable processor-based resource. They may be, but are not limited to, a central processing unit ("CPU"), a hardware microprocessor, a multi-core processor, a single core processor, a field programmable gate array ("FPGA"), a controller, a microcontroller, an application specific integrated circuit ("ASIC"), a digital signal processor ("DSP"), or other similar processing device capable of executing any type of instructions, algorithms, or software for controlling the operation and performing the functions of physiological monitoring device 102. In some embodiments, the one or more processors 110 may comprise a processor chipset including, for example and without limitation, one or more co-processors.

The display/GUI 112 may be configured to display various patient data, sensor data, and hospital or patient care information, and includes a user interface implemented for allowing interaction and communication between a user and physiological monitoring device 102. The display/GUI 112 may include a keyboard (not shown) and/or pointing or tracking device (not shown), as well as a display, such as a liquid crystal display ("LCD"), cathode ray tube ("CRT") display, thin film transistor ("TFT") display, light-emitting diode ("LED") display, high definition ("HD") display, or other similar display device that may include touch screen capabilities. The display/GUI 112 may provide a means for inputting instructions or information directly to the physiological monitoring device 102. The patient information displayed may, for example, relate to the measured physiological parameters of the patient 106 (e.g., ECG readings).

The communications interface 114 may permit the physiological monitoring device 102 to directly or indirectly (via, for example, a monitor mount) communicate with one or more computing networks and devices, workstations, consoles, computers, monitoring equipment, alert systems, and/or mobile devices (e.g., a mobile phone, tablet, or other hand-held display device). The communications interface 114 may include various network cards, interfaces, communication channels, cloud services, encryption, antennas, and/or circuitry to permit wired and wireless communications with such computing networks and devices. The communications interface 114 may be used to implement, for example, a BLUETOOTH® connection, a cellular network connection, and/or a WIFI® connection with such computing networks and devices. Example wireless communication connections implemented using the communication interface 114 include wireless connections that operate in accordance with, but are not limited to, IEEE802.11 protocol, a Radio Frequency For Consumer Electronics ("RF4CE") protocol, and/or IEEE802.15.4 protocol (e.g., ZigBee® protocol). In essence, any wireless communication protocol may be used.

Additionally, the communications interface 114 may permit direct (i.e., device-to-device) communications (e.g., messaging, signal exchange, etc.) such as from a monitor mount to physiological monitoring device 102 using, for example, a universal serial bus ("USB") connection or other communication protocol interface. The communication interface 114 may also permit direct device-to-device connection to other devices such as to a tablet, computer, or similar electronic device; or to an external storage device or memory.

The memory 116 may be a single memory device or one or more memory devices at one or more memory locations that may include, without limitation, one or more of a random-access memory ("RAM"), a memory buffer, a hard drive, a database, an erasable programmable read only memory ("EPROM"), an electrically erasable programmable read only memory ("EEPROM"), a read only memory ("ROM"), a flash memory, hard disk, various layers of memory hierarchy, or any other non-transitory computer readable medium. The memory 116 may be on-chip or off-chip depending on the implementation of the one or more processors 110. The memory 116 may be used to store any type of instructions and patient data associated with algorithms, processes, or operations for controlling the general functions and operations of the physiological monitoring device 102.

The power source 118 may include a self-contained power source such as a battery pack and/or include an interface to be powered through an electrical outlet, either directly or by way of a monitor mount. The power source 118 may also be a rechargeable battery that can be detached allowing for replacement. In the case of a rechargeable battery, a small built-in back-up battery (or super capacitor) can be provided for continuous power to be provided to the physiological monitoring device 102 during battery replacement. Communication between the components of the physiological monitoring device 102 in this example (may be established using the internal bus 119.

The physiological monitoring device 102 may be attached to one or more of several different types of sensors 104 and may be configured to measure and readout physiological data related to patient 106. As noted, the sensors 104 may be attached to the physiological monitoring device 102 by the sensor and cable assemblies 120 which may be, for example, cables coupled to sensor interface 108. Additionally, or alternatively, one or more sensors 104 may be connected to sensor interface 108 via a wireless connection. In which case sensor interface 108 may include circuity for receiving data from and sending data to one or more devices using, for example, a WIFI® connection, a cellular network connection, and/or a BLUETOOTH® connection.

The data signals received from the sensors 104 may be analog signals. For example, the data signals for the ECG may be input to the sensor interface 108, which can include an ECG data acquisition circuit (not shown separately in FIG. 1). An ECG data acquisition circuit may include amplifying and filtering circuity as well as analog-to-digital (A/D) circuity that converts the analog signal to a digital signal using amplification, filtering, and A/D conversion methods. In the event that the ECG sensor is a wireless sensor, the sensor interface 108 may receive the data signals from a wireless communication module (not shown in FIG. 1). Thus, the sensor interface 108 is a component which may be configured to interface with the one or more sensors 104 and receive sensor data therefrom.

As further described herein, the processing performed by an ECG data acquisition circuit may generate analog data waveforms or digital data waveforms that are analyzed by, in this particular embodiment, a microcontroller. However, other embodiments may use other kinds of processors disclosed above. The microcontroller may be one of the processors 110.

The one or more processors 110, for example, may analyze the ECG waveforms to identify certain waveform characteristics and threshold levels indicative of conditions (abnormal and normal) of the patient 106 using one or more monitoring methods. A monitoring method may include comparing an analog or a digital waveform characteristic or an analog or digital value to one or more threshold values and generating a comparison result based thereon. The microcontroller may be, for example, a processor, an FPGA, an ASIC, a DSP, a microcontroller, or similar processing device.

The microcontroller may include a memory (an on-chip memory) or use a separate memory 116 (an off-chip memory). The memory may be, for example, a RAM, a memory buffer, a hard drive, a database, an EPROM, an EEPROM, a ROM, a flash memory, a hard disk, or any other non-transitory computer readable medium. The memory 116 may store software or algorithms with executable instructions and the microcontroller may execute a set of instructions of the software or algorithms in association with executing different operations and functions of the physiological monitoring device 102 such as analyzing the digital data waveforms related to the data signals from the sensors 104.

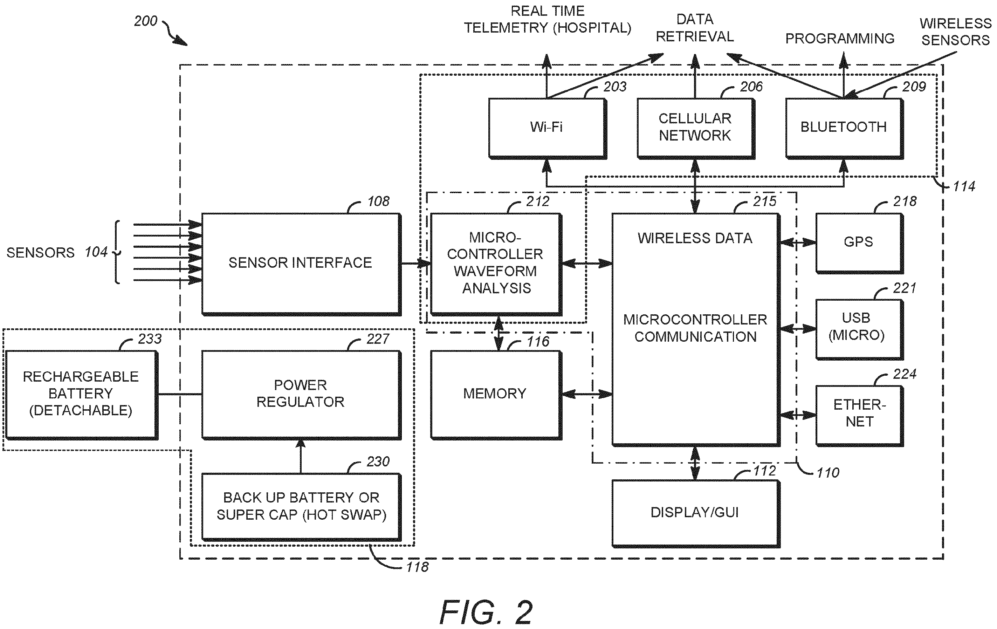

To further an understanding of this particular aspect of the claimed subject matter, FIG. 2 is a schematic diagram of one particular example of a physiological monitoring device 200 such as the physiological monitoring device 100 in FIG. 1. The physiological monitoring device 200 is, in this particular embodiment, attached to several different types of the sensors 104 (including electrodes or other similar devices) known in the art for gathering physiological data related to the patient 106 (e.g., as shown on the left side of FIG. 1). The sensors 104 are communicatively coupled to physiological monitoring device 200 by, for example, a wired connection input to the sensor interface 108.

It is contemplated by the present disclosure that the physiological monitoring device 200 can also be connected to other wired sensors (not shown) or to other wireless sensors (not shown) using the communication interface 114 of FIG. 1. The communication interface 114 in these embodiments includes circuity for receiving data from and sending data to one or more devices using, for example, a BLUETOOTH® connection 209. The communications interface 114 shown in FIG. 1 is represented in FIG. 2 by the combination of microcontroller 212 and elements 203-209.

The data signals from the sensors 104 received by the physiological monitoring device 200 include data related to, for example, an ECG, SpO2, NIBP, temperature, and/or etCO2. The data signals received from an ECG sensor and the SpO2 sensor can be analog signals. The data signals for the ECG and the SpO2 are input to the sensor interface 108, which can include an ECG data acquisition circuit and a SpO2 data acquisition circuit. Both the ECG data acquisition circuit and the SpO2 data acquisition circuit include amplifying and filtering circuity as well as analog-to-digital (A/D) circuity that convert the analog signal to a digital signal using amplification, filtering, and A/D conversion methods known in the art.

As another example, the data signals related to NIBP, temperature, and etCO2 can be received from the sensors 104 to the sensor interface 108, which can include a physiological parameter interface such as serial interface circuitry for receiving and processing the data signals related to NIBP, temperature, and etCO2. The ECG data acquisition circuit, an SpO2 data acquisition circuit, and a physiological parameter interface are described as part of the sensor interface 108. However, it is contemplated by the present disclosure that the ECG data acquisition circuit, the SpO2 data acquisition circuit, and the physiological parameter interface can be implemented as circuits separate from the sensor interface 108.

The processing performed by the ECG data acquisition circuit, the SpO2 data acquisition circuit, and external physiological parameter interface produces digital data waveforms that are analyzed by the microcontroller 212. The processors 3 shown in FIG. 1 are represented in FIG. 2 as microcontrollers 212 and 215. The microcontroller 212, for example, analyzes the digital waveforms to identify certain digital waveform characteristics and threshold levels indicative of conditions (abnormal and normal) of the patient 106 using methods known in the art. The microcontroller 212 includes a memory or uses the memory 116.

The memory stores software or algorithms with executable instructions and the microcontroller 212 can execute a set of instructions of the software or algorithms in association with executing different operations and functions of the physiological monitoring device 200 such as analyzing the digital data waveforms related to the data signals from the sensors 104. The results of the operations performed by the microcontroller 212 are passed to the microcontroller 215. The microcontroller 215 includes a memory or uses the memory 116.

As noted above, in FIG. 2, the communication interface 114 shown in FIG. 1 is represented by the combination of microcontroller 215 and elements 203-224. For example, the microcontroller 215 includes communication interface circuitry for establishing communication connections with various devices and networks using both wired and wireless connections, and transmitting physiological data, patient and transport information (e.g., transport times and patient location information), results of the analysis by the microcontroller 212, and alerts and/or alarms to the patient 106, clinicians and/or caregivers. The memory 116 stores software or algorithms with executable instructions and the microcontroller 215 can execute a set of instructions of the software or algorithms in association with establishing the communication connections.

As shown in FIG. 2, wireless communication connections established by the communication interface circuity of microcontroller 215 include a BLUETOOTH® connection 209, a cellular network connection 206, and a WIFI® connection 203. The wireless communication connections can allow, for example, patient and hospital information, alerts, and physiological data to be transmitted in real-time within a hospital wired or wireless communications network (e.g., WIFI®, Ethernet) as well as allow for patient and hospital information, alerts, and physiological data to be transmitted in real-time to other devices (e.g., BLUETOOTH® 209 and/or cellular networks 206).

It is also contemplated by the present disclosure that the communication connections established by the microcontroller 215 permit communications over other types of wireless networks using alternate hospital wireless communications such as wireless medical telemetry service ("WMTS"), which can operate at specified frequencies (e.g., 1.4 GHz). Other wireless communication connections can include wireless connections that operate in accordance with, but are not limited to, IEEE802.11 protocol, a Radio Frequency For Consumer Electronics (RF4CE) protocol, ZigBee protocol, and/or IEEE802.15.4 protocol.

The BLUETOOTH® connection 209 can also be used to provide the transfer of data to a nearby device (e.g., tablet) for review of data and/or changing of operational settings of the physiological monitoring device 200. The microcontroller 215 of the physiological monitoring device 200 provides a communication connection by direct wired (e.g., hard-wired) connections for transferring data using, for example, a USB connection 221 to a tablet, PC, or similar electronic device (not shown); or using, for example, an ethernet connection 224 to an external storage device or memory. Additionally, the microcontroller 215 includes a connection to a display/GUI 112 including a GUI for displaying patient information, physiological data or measured data, measurement schedules, alerts or alarms for the patient, clinicians and/or caregiver's information. Although the physiological monitoring device 200 is described in FIG. 1 as having two microcontrollers 212 and 215, it is contemplated by the disclosure of the present application that one microcontroller can be implemented to perform the functions of the two microcontrollers 212 and 215.

As shown in FIG. 2, the physiological monitoring device 200 includes a global positioning system (GPS) or other location data system 218 that can be connected to the communication interface circuity of microcontroller 215 so that the physiological monitoring device can transmit to the clinician, caregiver, or other devices the location of the patient 106 at all times including the location of the patient 106. Additionally, the location of the patient 106 can be used by the microcontroller 215 to determine an estimated time of arrival of the patient 106.

For example, location data provided by the location data system 218, which may include information on a floor level, can be compared to stored information related to a hospital layout or a hospital map as well as information related to a patient's scheduled care (e.g., treatment or procedure scheduled for the patient 106 in a patient care area within the hospital). Based on the comparison results, the microcontroller 215 can determine the estimated time of arrival of the patient 106 to the patient care area within the hospital. The estimated time of arrival can be transmitted by the communication interface circuity of microcontroller 215 to, for example, the hospital wireless communications system.

Additionally, if it is determined by the microcontroller 215 that the patient 106 is not within the vicinity of the hospital wireless communications system (e.g., based on input from the location data system 218), the pertinent physiological data can be recorded and stored in the memory 116. Additionally, if the BLUETOOTH® connection 209 or WIFI® connection 203 are not available (e.g., out of transmission range or not operable), then the microcontroller can store the physiological data in the memory 116 for later transmission when the BLUETOOTH® connection or WIFI® connection becomes available.

The power source 118 shown in FIG. 1 is represented by elements 227-233 in FIG. 2. As shown in FIG. 2, the power can be supplied using a rechargeable battery 233 that can be detached allowing for replacement. The rechargeable battery 233 may be, for example, a rechargeable lithium-ion battery. Additionally, a small built-in backup battery 230 (or supercapacitor) is provided for continuous power to the physiological monitoring device 200 during battery replacement. A power regulator or regulation circuit 227 is provided between the rechargeable battery 233 and small backup battery 230 to control which battery provides power to the physiological monitoring device 200.

It is also contemplated by the present disclosure that the power regulator 227 can include a self-contained power source such as a battery pack and/or include an interface to be powered through an electrical outlet (either directly or by way of a monitor mount). Communication between the components of the physiological monitoring device 200 can be established using an internal bus similar to the internal bus 119 discussed with reference to FIG. 1.

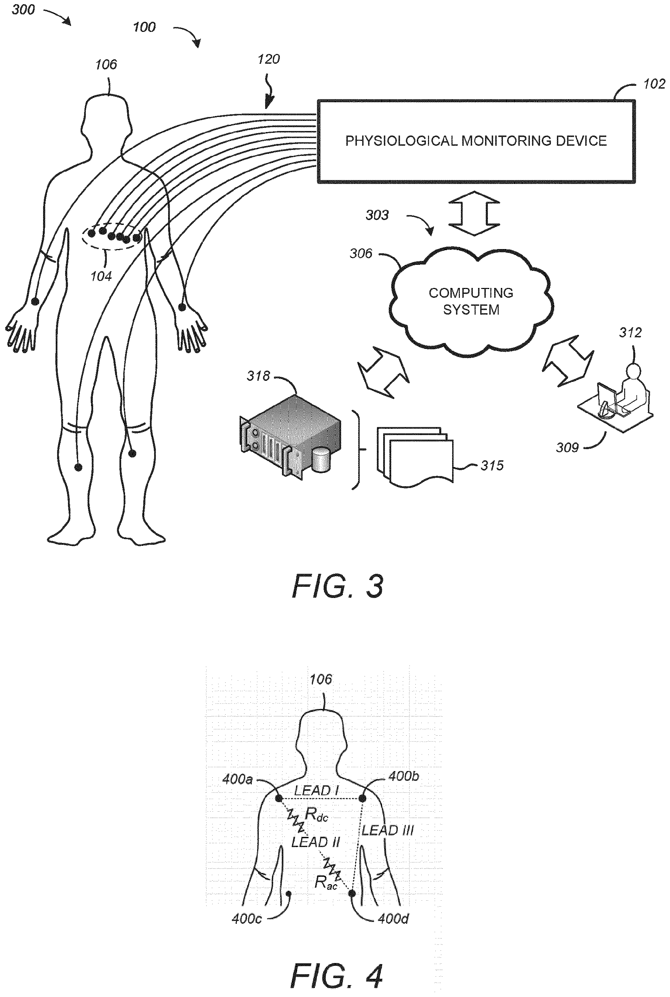

The physiological monitoring system 100 may also be deployed within the context of a larger patient monitoring system 300, shown in FIG. 3. FIG. 3 is a schematic diagram of an example of a patient monitoring system 300 including a computing system 303 and the physiological monitoring system 100 of FIG. 1 according to an embodiment of the present disclosure. The computing system 303 may be a distributed computing environment 306, including a network and/or cloud services, over which the physiological monitoring system 100 pushes and/or pulls data and information. The communications among the physiological monitoring device 102, the computing system 306, and other resources of the patient monitoring system 300 may be either wireless or wired depending upon the technical capabilities of the various components.

For example, the facility (not otherwise shown) in which the patient 106 is located may include a central monitoring station 309 from which a caregiver 312 may monitor the physical condition of multiple patients 106 concurrently. The physiological monitoring device 102, using, for example, the communications interface 114 shown in FIG. 1, may push sensed physiological parameters and/or signals representative thereof to the central monitoring station 309 over the computing system 306. The pushed data and information may then be displayed for the caregiver 312 for review and consideration.

For another example, the physiological monitoring device 100 may pull information from an electronic medical record 315 for the patient 106 from a records repository 318 using, for example, the communications interface 114 shown in FIG. 1. The pulled information can then be displayed by the physiological monitoring device 102. Such pulled information might include, depending on the implementation and without limitation, medication regimens, diagnosed medical conditions, medical history, historical medical data, etc. Similarly, data sensed by or entered at the physiological monitoring device 102 may be pushed to, for instance, the ERM 315 for storage and later retrieval.

FIG. 4 presents a patient model illustrating these measurement and acquisition of the impedance respiration signals in the embodiments disclosed herein. Note that alternative embodiments may use different patient models than the one shown in FIG. 4. Note also that the placement of the four sensors 400a-400d using three "leads" is determined so as to measure the thoracic resistance through the chest of the patient 106. Note that the term "lead" as used in this context does not refer to a sensor and cable assembly, but rather to the voltage potential between two of the sensors 400a-400d.

$R_{dc}$ is the constant thoracic impedance while $R_{ac}$ is the measured change to impedance due to breathing. More particularly, in order to measure the change in impedance caused by breathing, impedance respiration measurement uses the ECG electrodes that inject a weak, high frequency square wave current (~70 nA, 40 kHz) into the patient 106 using one of three lead selections, namely Lead I, Lead II, or Lead III. The voltage across the sensors 400a-400d is measured from which the impedance signal is derived. The impedance signal then undergoes hardware and software filtering, as described below.

Those in the art having the benefit of this disclosure will appreciate that lead selection will depend, at least to some degree, on the physiological parameter being determined. This is because the ECG signal should reflect that physiological parameter as accurately or strongly as possible from among the available ECG signals. Hence, in the illustrated embodiments, the leads indicated in FIG. 4 are preferred since it is the impedance respiration that is determined.

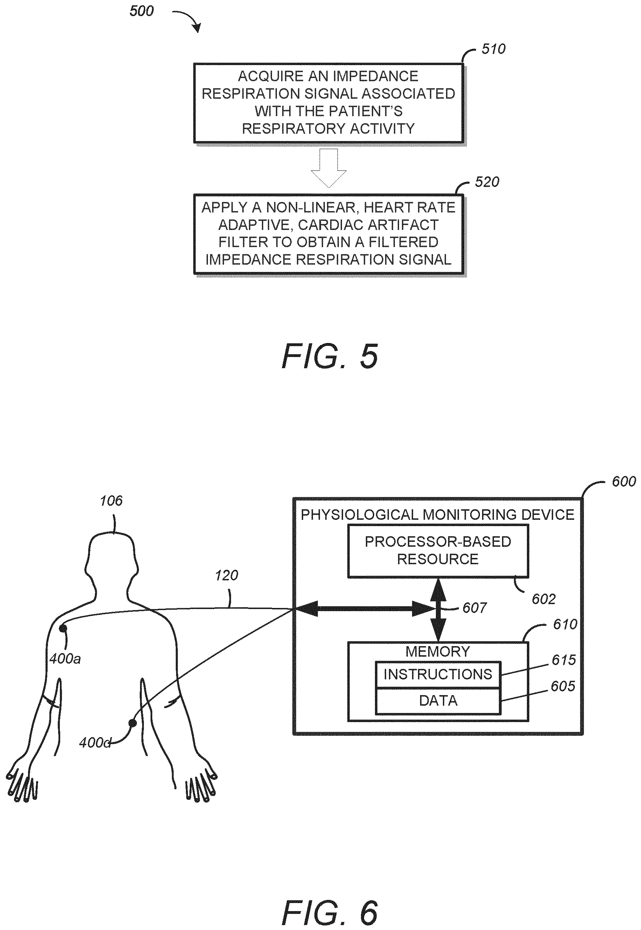

FIG. 5 illustrates a method 500 for use in quantifying a physiological parameter of a monitored patient in accordance with one or more embodiments. FIG. 6 schematically depicts one particular implementation of a physiological monitoring device 600. The depiction of the physiological monitoring device 600 is simplified from what is shown in FIG. 1 and FIG. 2 so as not to obscure those aspects of the claimed subject matter now under discussion. More particularly, as illustrated in FIG. 6, a processor-based resource 602 communicates with a memory 610 over an internal bus system 607. Thus, some of the details discussed relative to and shown in FIG. 1 and FIG. 2 have been omitted for purposes of this discussion although such details will be pertinent in most embodiments.

The processor-based resource 602 may be, for example, one of the one or more processors 110 shown in FIG. 1, including one or both of the microcontrollers 212, 215 shown in FIG. 2. The processor-based resource 602 operates under programmed control of the instructions 615. The processor-based resource 602 therefore communicates with the memory 610 over the bus system 607 to, among other things, execute the instructions 615 stored thereon.

The execution of the instructions may cause the processor-based resource 602 to perform certain tasks such as some portions of the method 500 outlined in FIG. 5. However, depending on the environment, the processor-based resource 602 may also perform other tasks such as pushing data to other computing resources, transmitting alarms, *etc.* Thus, in this sense, the processor-based resources 602, as well as the physiological monitoring device 600 as a whole, may be considered to be "programmed" or "configured" to perform certain functions as described herein.

Referring now to FIG. 5 and FIG. 6 collectively, the method 500 begins by acquiring (at 510) an impedance respiration signal associated with the patient's respiratory activity. As is shown, the impedance respiration signal is acquired by performing an ECG, represented by the single and cable assembly 120 and then processing the acquired ECG signal using the processor-based resource 602 of the physiological monitoring device 600. The acquired ECG signal may be buffered in as data 605 in the memory 610 or processed in real time. Similarly, the acquired impedance respiration signal, once processed, may be buffered as data 605 in the memory 610 as needed or desired.

Accordingly, the ECG signal and the impedance respiration signal may be processed, displayed, or otherwise handled or manipulated in real time or near real time. The term "near real time" means as closed to real time as the computing resources of a given embodiment permit. Some embodiments may also store the ECG signal, impedance respiration signal, or other data in the memory 610 longer term. For example, some embodiments may manage processing resources by storing data 605 before pushing it out as described above. Similarly, some embodiments may store data 605 pulled from other locations as described above.

The method 500 then continues by applying (at 520) a non-linear, heart rate adaptive, cardiac artifact filter to obtain a filtered impedance respiration signal. One particular non-linear, heart rate adaptive, cardiac artifact filter is described further below. The filtered impedance respiration signal, like the acquired ECG signal and the impedance respiration signal may be buffered or stored as data 605 in the memory 610. That is, the filtered impedance respiration signal may be processed, displayed, or otherwise handled or manipulated in real time or near real time.

Further processing and handling beyond the method 500 may be performed in various embodiments. For example, in embodiments wherein the physiological monitoring device 600 includes a display (*e.g.*, the display/GUI 112 in FIG. 1), one or more of the acquired ECG signal, the impedance respiration signal, and/or the filtered impedance respiration signal may be displayed. Some embodiments may analyze the filtered impedance respiration signal. For example, the filtered impedance respiration signal may be analyzed for the presence of an alarm condition and, upon detecting an alarm condition, issuing an alarm. Such an alarm may be visual and presented on a display or auditory, presuming the physiological monitoring device is suitably implemented, or a combination thereof. Those in the art having the benefit of this disclosure may appreciate still other variations.

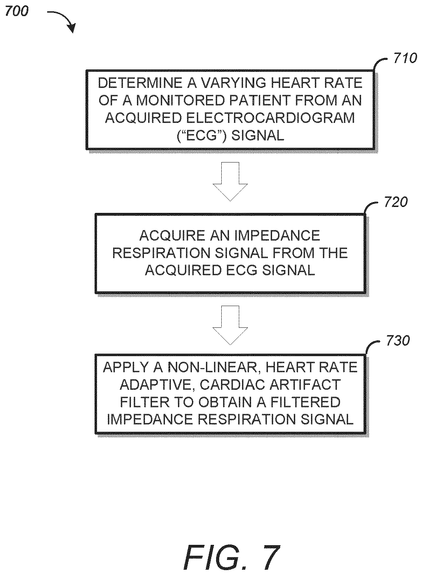

FIG. 7 illustrates method for non-linear, heart rate adaptive, cardiac artifact filtering an impedance respiration signal in accordance with various embodiments disclosed herein. The method 700 begins by determining (at 710) a varying heart rate of a monitored patient from an acquired electrocardiogram ("ECG") signal and acquiring (at 720) an impedance respiration signal. Note that these two acts may be performed in either order or even in parallel. The method 700 then removes (at 730) a frequency component from the impedance respiration signal, the frequency component corresponding to the varying heart rate, to smooth the impedance respiration signal and filter out at least one cardiac artifact.

Expressed more mathematically, consider an input signal $x(n)$ that has cardiac artifact superimposed on it (*e.g.*, an impedance respiration signal). Such an input signal may be represented as Eq. (1) below, in which $x(n)$ is the acquired respiration signal, $y(n)$ is the true impedance respiration signal, and $e(n)$ is the cardiac artifact noise at a discrete timestamp $n$. Assuming that the cardiac artifact noise is sinusoidal with a constant amplitude $A$, a filter can be designed that can learn this noise $e(n)$ and subtract it out.

$$x(n) = y(n) + e(n) \qquad (1)$$

The noise is first estimated based on a sine wave and expressed in digital signal as Eq. (2) below. In Eq. (2), $\omega$ is the noise frequency (e.g., derived from varying heart rate obtained from the acquired ECG signal), $n$ is the nth sampled data point, and $T$ is the sampling period. Note that the heart rate can be extracted from the acquired ECG signal using any suitable technique known to the art.

$$e(nT) = A \sin \omega nT \qquad (2)$$

The noise estimate for the previous and the next sampled data point can be respectively expressed Eq. (3), below, and Eq. (4), below, respectively.

$$e(nT - T) = A \sin(\omega nT - \omega T) \qquad (3)$$

$$e(nT + T) = A \sin(\omega nT + \omega T) \qquad (4)$$

The future noise estimate in terms of the past two noise estimates can be then expressed as Eq. (5), below, using the trigonometric identity in Eq. (6).

$$e(nT + T) = 2A\sin(\omega nT)\cos(\omega T) - A\sin(\omega nT - \omega T) \qquad (5)$$

$$\sin(a + b) = 2\sin(a)\cos(b) - \sin(a - b) \qquad (6)$$

Since for a given noise frequency and sampling period $T$ cos $(\omega T)$ is equal to a constant $N$ it can be substituted in Eq. (5) along with Eq. (3) and Eq. (4) to yield Eq. (7).

$$e(nT + T) = 2Ne(nT) - e(nT - T) \qquad (7)$$

Eq. (7) uses the present estimate of noise $e(nT)$ together with the past estimate $e(nT-T)$ to predict the next noise estimate. If the noise prediction is correct, the next noise estimate should be equal to the actual noisy input $x(nT+T)$ except for some direct current ("DC") offset. Any DC offset can be approximated by the difference between $e(nT)$ and $x(nT)$ and subtracted out. The error estimate is computed as Eq. (8) below.

$$f(nT + T) = (x(nT + T) - e(nt + T)) - (x(nT) - e(nT)) \qquad (8)$$

If this function in Eq. (8) is zero, the noise estimate is correct. If the function is not zero an adjustment is applied before estimating future points. This filtering adjustment factor is an empirically determined value. A small constant filtering adjustment factor results in a lower degree of filtering a large constant will perform higher filtering but can also add more noise to the output especially when the amplitude of the true respiration signal is small.

A solution to fix this is made by using an adaptive value of the filtering adjustment factor based on a scaled value of the error estimate $d$ and is limited to a maximum value of 1. The adjusted noise estimate is then expressed as Eq. (9).

$$e(nT + T) = e(nT + T) + \text{sign}(d) * \min\left(1, \frac{abs(d)}{10}\right) \qquad (9)$$

After the Adjustment is applied the output of the filter for a given time stamp is computed as Eq. 10.

$$y(nT + T) = x(nT + T) - e(nT + T) \qquad (10)$$

Note that filtering of cardiac artifact from the respiration signal $N=\cos(\omega T)$ in Eq. (7) depends on the frequency of the cardiac artifact and thus the equation can be expressed as Eq. (11), where $f_{HR}$ is the heart rate in Hz and $f_s$ is the sampling rate of the impedance respiration signal.

$$e(n + 1) = 2 * \cos\left(2 * pi * \frac{f_{HR}}{f_s}\right) * e(n) - e(n - 1) \qquad (11)$$

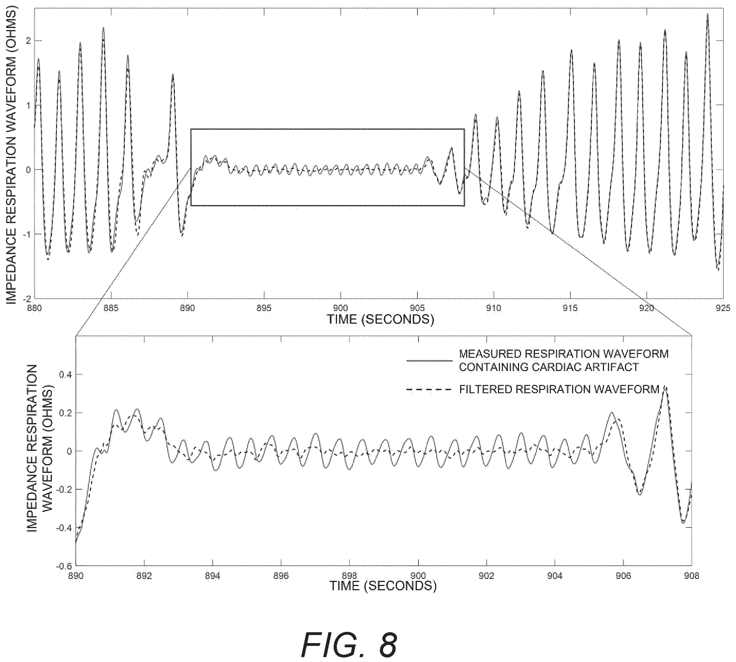

Thus, the technique essentially learns how much cardiac artifact is present and subtracts it from the signal leaving only the respiration portion of the signal. FIG. 8 shows the filtering effect when applied to real patient data. This plot shows the measured impedance respiration waveform (solid line) and the filtered impedance respiration waveform (dashed line) overlaid on top of it. The fluctuations due to the cardiac artifact are reduced in the filtered signal. With sufficient attenuation, the respiration rate now stays close to the true respiration rate instead of the true heart rate.

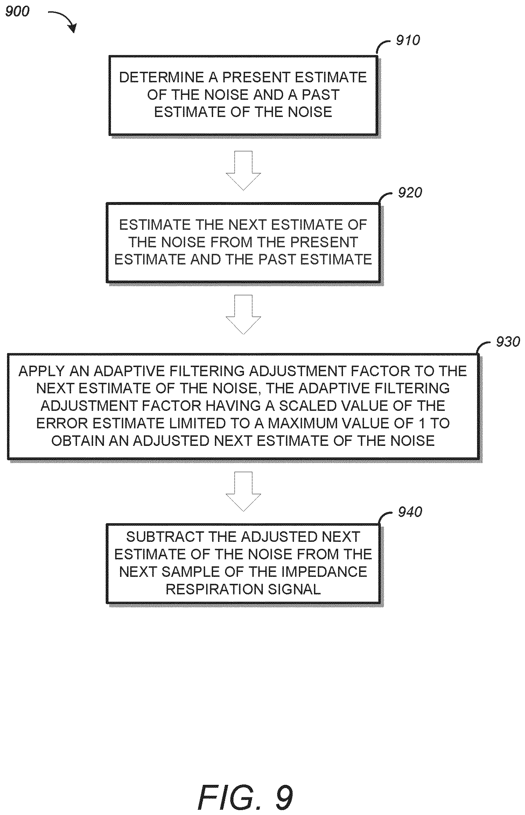

FIG. 9 illustrates one particular embodiment for removing the frequency component from the impedance respiration signal. In this embodiment, the frequency component corresponding to the varying heart rate constitutes noise in the impedance respiration signal. In this embodiment, the method 900 removes the frequency component from the impedance respiration signal on a sample-by-sample basis. The method 900 begins by determining (at 910) a present estimate of the noise and a past estimate of the noise. The method 900 then estimates (at 920) the next estimate of the noise from the present noise estimate and the part noise estimate. Next, the method 900 applies (at 930) an adaptive filtering adjustment factor to the next estimate of the noise, the adaptive filtering adjustment factor having a scaled value of the error estimate limited to a maximum value of 1 to obtain an adjusted next estimate of the noise. The method 900 then subtracts (at 940) the adjusted next estimate of the noise from the next sample of the impedance respiration signal.

Accordingly, as disclosed above, the present disclosure provides a non-linear, heart rate adaptive, cardiac artifact filter designed to reduce cardiac artifacts in a patient's impedance respiration signal. The patient's impedance respiration signal may be acquired using, for example, ECG sensors. The adaptive cardiac filter can be used to filter unwanted cardiac artifacts from the impedance respiration signal to provide a more accurate representation of the patient's respiratory activity. The filtered impedance respiration signal can then be displayed or analyzed to further the monitoring and treatment of the patient.

Reducing the level of cardiac artifacts in the impedance respiration signal as described herein will provide a more accurate measurement of the patient's respiration rate when cardiac artifacts are present. As described above, cardiac artifacts may be responsible for false high respiration rates or cause a computed respiration rate to be falsely measured to be approximately equal to the heart rate of the subject. Falsely detected breaths could also lead to missed apnea events. Application of the technique described herein may therefore mitigate one or more of these concerns to improve monitoring of the patient's physical condition.

Thus, in a first embodiment, a method for non-linear, heart rate adaptive, cardiac artifact filtering an impedance respiration signal comprises: determining a varying heart rate of a monitored patient from an acquired electrocardiogram ("ECG") signal; acquiring an impedance respiration signal; and removing a frequency component from the impedance respiration signal, the frequency component corresponding to the varying heart rate, to smooth the impedance respiration signal and filter out at least one cardiac artifact.

In a second embodiment, the frequency component in the first embodiment corresponds to the varying heart rate constitutes noise in the impedance respiration signal; and removing the frequency component from the impedance respiration signal comprises, on a sample-by-sample basis: determining a present estimate of the noise and a past estimate of the noise; estimating the next estimate of the noise from the present estimate and the past estimate; applying an adaptive filtering adjustment factor to the next estimate of the noise, the adaptive filtering adjustment factor having a scaled value of the error estimate limited to a maximum value of 1 to obtain an adjusted next estimate of the noise; and subtracting the adjusted next estimate of the noise from the next sample of the impedance respiration signal.

In a third embodiment, the adjusted next noise estimate in the first embodiment is mathematically represented as:

$$e(nT + T) = e(nT + T) + \text{sign}(d) * \min\left(1, \frac{abs(d)}{10}\right)$$

where:

$n \equiv$ the sample number;
$T \equiv$ the sampling period; and
$d \equiv$ the error estimate.

In a fourth embodiment, the adjusted next noise estimate in the first embodiment is mathematically represented as:

$$e(n + 1) = 2 * \cos\left(2 * pi * \frac{f_{HR}}{f_s}\right) * e(n) - e(n - 1)$$

where:

$n \equiv$ the sample number;
$f_{HR} \equiv$ the patient's heart rate in Hz; and
$f_s \equiv$ the sampling rate of the respiration signal.

In a fifth embodiment, in the method of the fourth embodiment, $f_{HR}$=0.25-5 Hz.

In a sixth embodiment, the method of the first embodiment further comprises analyzing the filtered impedance respiration signal for patient conditions.

In a seventh embodiment, in the method of the sixth embodiment, analyzing the filtered impedance respiration signal includes detecting an alarm condition; and the method further comprises issuing an alarm.

In an eighth embodiment, a method for monitoring a patient's physical condition, the method comprises: acquiring an impedance respiration signal associated with the patient's cardiac activity; and applying a non-linear, heart rate adaptive, cardiac artifact filter to obtain a filtered impedance respiration signal.

In a ninth embodiment, the method of eighth embodiment further comprises displaying the filtered impedance respiration signal.

In a tenth embodiment, the method of the ninth embodiment further comprises analyzing the filtered impedance respiration signal.

In an eleventh embodiment, in the method of the tenth embodiment, analyzing the filtered impedance respiration signal includes detecting an alarm condition; and the method further comprises issuing an alarm.

In a twelfth embodiment, the method of eighth embodiment further comprises: analyzing the filtered impedance respiration signal includes to detect an alarm condition; and issuing an alarm.

In a thirteenth embodiment, in the method of eighth embodiment, acquiring the impedance respiration signal includes performing an electrocardiogram ("ECG").

In a fourteenth embodiment, applying the non-linear, heart rate adaptive, cardiac artifact filter to obtain a filtered impedance respiration signal in the eighth embodiment includes any one of the methods of first through seventh embodiments.

In a fifteenth embodiment, a physiological monitoring device comprises: a processor-based resource; and a memory encoded with instructions that, when executed by the processor-based resource, performs any one of the methods of first through seventh embodiments.

In a sixteenth embodiment, the physiological monitoring device of the fifteenth embodiment further comprising a display and the method further comprises displaying the filtered impedance respiration signal.

In a seventeenth embodiment, in the physiological monitoring device of the fifteenth embodiment the method further comprises any one of the methods of the tenth to the twelfth embodiments.

In an eighteenth embodiment, a system for physiologically monitoring a patient comprises: a plurality of electrocardiogram ("ECG") sensors; and a physiological monitoring device communicating with the plurality of ECG system and performing any one of the methods of the eighth to fourteenth embodiments.

In a nineteenth embodiment, the system of the eighteenth embodiment further comprises: a computing system communicating with the physiological monitoring device; and a records repository further comprising a plurality of electronic medical records ("ERMs") communicating with the physiological monitoring device through the computing system. The physiological monitoring device: pushes information to at least one of the ERMs; and pulls information from at least one of the ERMs, which may be the same or different from the ERM to which the physiological monitoring device pushes information.

In a twentieth embodiment, the system of the nineteenth embodiment further comprises a central monitoring station communicating with the physiological monitoring device through the computing system from which a caregiver monitors the patient's condition from the central monitoring station.

In a twenty-first embodiment, the system of the eighteenth embodiment further comprises a computing system communicating with the physiological monitoring device; and a central monitoring station communicating with the physiological monitoring device through the computing system. A caregiver monitors the patient's condition from the central monitoring station.

In a twenty-second embodiment a method for non-linear, heart rate adaptive, cardiac artifact filtering is substantially as shown and described herein.

In a twenty-third embodiment, a non-linear, heart rate adaptive, cardiac artifact filter is substantially as shown and described.

In a twenty-fourth embodiment, a method for monitoring a patient's physical condition is substantially as shown and described.

In a twenty-fifth embodiment, a physiological monitoring device is substantially as shown and described.

In a twenty-sixth embodiment, a system for physiologically monitoring a patient is substantially as shown and described.

The expressions such as "include" and "may include" which may be used in the present disclosure denote the presence of the disclosed functions, operations, and constituent elements, and do not limit the presence of one or more additional functions, operations, and constituent elements. In the present disclosure, terms such as "include" and/or "have", may be construed to denote a certain characteristic, number, operation, constituent element, component or a combination thereof, but should not be construed to exclude the existence of or a possibility of the addition of one or more other characteristics, numbers, operations, constituent elements, components or combinations thereof.

As used herein, the article "a" is intended to have its ordinary meaning in the patent arts, namely "one or more." Herein, the term "about" when applied to a value generally means within the tolerance range of the equipment used to produce the value, or in some examples, means plus or minus 10%, or plus or minus 5%, or plus or minus 1%, unless otherwise expressly specified. Further, herein the term "substantially" as used herein means a majority, or almost all, or all, or an amount with a range of about 51% to about 100%, for example. Moreover, examples herein are intended to be illustrative only and are presented for discussion purposes and not by way of limitation.

As used herein, to "provide" an item means to have possession of and/or control over the item. This may include, for example, forming (or assembling) some or all of the item from its constituent materials and/or, obtaining possession of and/or control over an already-formed item.

Unless otherwise defined, all terms including technical and/or scientific terms used herein have the same meaning as commonly understood by one of ordinary skill in the art to which the present disclosure pertains. In addition, unless otherwise defined, all terms defined in generally used dictionaries may not be overly interpreted. In the following, details are set forth to provide a more thorough explanation of the embodiments. However, it will be apparent to those skilled in the art that embodiments may be practiced without these specific details. In other instances, well-known structures and devices are shown in block diagram form or in a schematic view rather than in detail in order to avoid obscuring the embodiments. In addition, features of the different embodiments described hereinafter may be combined with each other, unless specifically noted otherwise. For example, variations or modifications described with respect to one of the embodiments may also be applicable to other embodiments unless noted to the contrary.

Further, equivalent or like elements or elements with equivalent or like functionality are denoted in the following description with equivalent or like reference numerals. As the same or functionally equivalent elements are given the same reference numbers in the figures, a repeated description for elements provided with the same reference numbers may be omitted. Hence, descriptions provided for elements having the same or like reference numbers are mutually exchangeable.

It will be understood that when an element is referred to as being "connected" or "coupled" to another element, it can be directly connected or coupled to the other element or intervening elements may be present. In contrast, when an element is referred to as being "directly connected" or "directly coupled" to another element, there are no intervening elements present. Other words used to describe the relationship between elements should be interpreted in a like fashion (e.g., "between" versus "directly between," "adjacent" versus "directly adjacent," etc.).

In the present disclosure, expressions including ordinal numbers, such as "first", "second", and/or the like, may modify various elements. However, such elements are not limited by the above expressions. For example, the above expressions do not limit the sequence and/or importance of the elements. The above expressions are used merely for the purpose of distinguishing an element from the other elements. For example, a first box and a second box indicate different boxes, although both are boxes. For further example, a first element could be termed a second element, and similarly, a second element could also be termed a first element without departing from the scope of the present disclosure.

A sensor refers to a component which converts a physical quantity to be measured to an electric signal, for example, a current signal or a voltage signal. The physical quantity may for example comprise electromagnetic radiation (e.g., photons of infrared or visible light), a magnetic field, an electric field, a pressure, a force, a temperature, a current, or a voltage, but is not limited thereto.

ECG signal processing, as used herein, refers to, without limitation, manipulating an analog signal in such a way that the signal meets the requirements of a next stage for further processing. ECG signal processing may include converting between analog and digital realms (e.g., via an analog-to-digital or digital-to-analog converter), amplification, filtering, converting, biasing, range matching, isolation and any other processes required to make a sensor output suitable for processing.

Use of the phrases "capable of," "capable to," "operable to," or "configured to" in one or more embodiments, refers to some apparatus, logic, hardware, and/or element designed in such a way to enable the use of the apparatus, logic, hardware, and/or element in a specified manner. Use of the phrase "exceed" in one or more embodiments, indicates that a measured value could be higher than a pre-determined threshold (e.g., an upper threshold), or lower than a pre-determined threshold (e.g., a lower threshold). When a pre-determined threshold range (defined by an upper threshold and a lower threshold) is used, the use of the phrase "exceed" in one or more embodiments could also indicate a measured value is outside the pre-determined threshold range (e.g., higher than the upper threshold or lower than the lower threshold). The subject matter of the present disclosure is provided as examples of apparatus, systems, methods, circuits, and programs for performing the features described in the present disclosure. However, further features or variations are contemplated in addition to the features described above. It is contemplated that the implementation of the components and functions of the present disclosure can be done with any newly arising technology that may replace any of the above-implemented technologies.

The detailed description is made with reference to the accompanying drawings and is provided to assist in a comprehensive understanding of various example embodiments of the present disclosure. Changes may be made in the function and arrangement of elements discussed without departing from the spirit and scope of the disclosure. Various embodiments may omit, substitute, or add various procedures or components as appropriate. For instance, features described with respect to certain embodiments may be combined in other embodiments. In addition, descriptions of well-known functions and constructions may be omitted for clarity and conciseness. Accordingly, those of ordinary skill in the art will recognize that various changes and modifications of the examples described herein can be made without departing from the spirit and scope of the present disclosure.

Various modifications to the disclosure will therefore be readily apparent to those skilled in the art, and the generic principles defined herein may be applied to other variations without departing from the spirit or scope of the present disclosure. Throughout the present disclosure the terms "example," "examples," or "exemplary" indicate examples or instances and do not imply or require any preference for the noted examples. Thus, the present disclosure is not to be limited to the examples and designs described herein but is to be accorded the widest scope consistent with the principles and novel features disclosed.

This disclosure includes the following numbered Paragraphs or "Paras":

Para 1. A method for non-linear, heart rate adaptive, cardiac artifact filtering an impedance respiration signal, the method comprising: determining a varying heart rate of a monitored patient from an acquired electrocardiogram ("ECG") signal; acquiring an impedance respiration signal; and removing a frequency component from the impedance respiration signal, the frequency component corresponding to the varying heart rate, to smooth the impedance respiration signal and filter out at least one cardiac artifact.

Para 2. The method of Para 1, wherein: the frequency component corresponding to the varying heart rate constitutes noise in the impedance respiration signal; and removing the frequency component from the impedance respiration signal comprises, on a sample-by-sample basis: determining a present estimate of the noise and a past estimate of the noise; estimating the next estimate of the noise from the present estimate and the past estimate; applying an adaptive filtering adjustment factor to the next estimate of the noise, the adaptive filtering adjustment factor having a scaled value of the error estimate limited to a maximum value of 1 to obtain an adjusted next estimate of the noise; and subtracting the adjusted next estimate of the noise from the next sample of the impedance respiration signal.

Para 3. The method of Para 1, wherein the adjusted next noise estimate is mathematically represented as:

$$e(nT + T) = e(nT + T) + \text{sign}(d) * \min\left(1, \frac{abs(d)}{10}\right)$$

where:

$n \equiv$ the sample number;
$T \equiv$ the sampling period; and
$d \equiv$ the error estimate.

Para 4. The method of Para 1, wherein the adjusted next noise estimate is mathematically represented as:

$$e(n + 1) = 2 * \cos\left(2 * pi * \frac{f_{HR}}{f_s}\right) * e(n) - e(n - 1)$$

where:

$n \equiv$ the sample number;
$f_{HR} \equiv$ the patient's heart rate in Hz; and
$f_s \equiv$ the sampling rate of the respiration signal.

Para 5. The method of Para 4, wherein fHR = 0.25-5 Hz.

Para 6. The method of Para 1, further comprising analyzing the filtered impedance respiration signal for patient conditions.

Para 7. The method of Para 6, wherein: analyzing the filtered impedance respiration signal includes detecting an alarm condition; and the method further comprises issuing an alarm.

Para 8. A method for monitoring a patient's physical condition, the method comprising: acquiring an impedance respiration signal associated with the patient's cardiac activity; and applying a non-linear, heart rate adaptive, cardiac artifact filter to obtain a filtered impedance respiration signal.

Para 9. The method of Para 8, further comprising displaying the filtered impedance respiration signal.

Para 10. The method of Para 9, further comprising analyzing the filtered impedance respiration signal.

Para 11. The method of Para 10, wherein: analyzing the filtered impedance respiration signal includes detecting an alarm condition; and the method further comprises issuing an alarm.

Para 12. The method of Para 8, further comprising: analyzing the filtered impedance respiration signal includes to detect an alarm condition; and issuing an alarm.

Para 13. The method of Para 8, wherein acquiring the impedance respiration signal includes an electrocardiogram ("ECG").

Para 14. The method of Para 8, wherein applying the non-linear, heart rate adaptive, cardiac artifact filter to obtain the filtered impedance respiration signal includes any one of the methods of Paras 1-7.

Para 15. A physiological monitoring device, comprising: a processor-based resource; and a memory encoded with instructions that, when executed by the processor-based resource, performs any one of the methods of Paras 1-7.

Para 16. The physiological monitoring device of Para 15, further comprising a display; and wherein the method further comprises displaying the filtered impedance respiration signal.

Para 17. The physiological monitoring device of Para 15, wherein the method further comprises any one of the methods of Paras 10-12.

Para 18. A system for physiologically monitoring a patient, the system comprising: a plurality of electrocardiogram ("ECG") sensors; and a physiological monitoring device communicating with the plurality of ECG system and performing any one of the methods of Paras 8-14.

Para 19. The system of Para 18, further comprising: a computing system communicating with the physiological monitoring device; and a records repository further comprising a plurality of electronic medical records ("ERMs") communicating with the physiological monitoring device through the computing system; wherein the physiological monitoring device: pushes information to at least one of the ERMs; and pulls information from at least one of the ERMs, which may be the same or different from the ERM to which the physiological monitoring device pushes information.

Para 20. The system of Para 19, further comprising: a central monitoring station communicating with the physiological monitoring device through the computing system; wherein a caregiver monitors the patient's condition from the central monitoring station.

Para 21. The system of Para 18, further comprising: a computing system communicating with the physiological monitoring device; and a central monitoring station communicating with the physiological monitoring device through the computing system; wherein a caregiver monitors the patient's condition from the central monitoring station.

Para 22. A method for non-linear, heart rate adaptive, cardiac artifact filtering substantially as shown and described.

Para 23. A non-linear, heart rate adaptive, cardiac artifact filter substantially as shown and described.

Para 24. A method for monitoring a patient's physical condition substantially as shown and described.

Para 25. A physiological monitoring device substantially as shown and described.

Para 26. A system for physiologically monitoring a patient substantially as shown and described.

Para 27. The physiological monitoring device of any one of Paras 15 to 17, further comprising:

analyzing the filtered impedance respiration signal includes detecting an alarm condition; and

issuing an alarm.

Para 28. The physiological monitoring device of any Paras 15 to 17 and 27, wherein the acquired impedance respiration signal comprises an electrocardiogram ("ECG") signal.

Para 29. A physiological monitoring device of any one of Paras 15 to 17, 27, or 28 wherein:

the acquired impedance respiration signal comprises an acquired electrocardiogram ("ECG") signal; and

applying the non-linear, heart rate adaptive, cardiac artifact filter to obtain the filtered impedance respiration signal includes:

determining a varying heart rate of a monitored patient from the acquired electrocardiogram ("ECG") signal;

acquiring an impedance respiration signal; and

removing a frequency component from the acquired impedance respiration signal, the frequency component corresponding to the varying heart rate, to smooth the impedance respiration signal and filter out at least one cardiac artifact.

Para 30. The physiological monitoring device of any one of Paras 15 to 17 and 27 to 29, further comprising:

analyzing the filtered impedance respiration signal to detect an alarm condition; and

issuing an alarm.

**Claims**

1. A method for monitoring a patient's physical condition, the method comprising:

receiving an acquired impedance respiration signal associated with the patient's cardiac activity; and
applying a non-linear, heart rate adaptive, cardiac artifact filter to obtain a filtered impedance respiration signal.

2. The method of claim 1, further comprising displaying the filtered impedance respiration signal.

3. The method of any one of the preceding claims, wherein the acquired impedance respiration signal comprises an electrocardiogram ("ECG") signal.

4. The method of any one of the preceding claims, wherein:

the acquired impedance respiration signal comprises an acquired electrocardiogram ("ECG") signal; and
applying the non-linear, heart rate adaptive, cardiac artifact filter to obtain the filtered impedance respiration signal includes:

determining a varying heart rate of a monitored patient from the acquired electrocardiogram ("ECG") signal; and
acquiring an impedance respiration signal; and
removing a frequency component from the acquired impedance respiration signal, the frequency component corresponding to the varying heart rate, to smooth the impedance respiration signal and filter out at least one cardiac artifact.

5. The method of claim 4, wherein:

the frequency component corresponding to the varying heart rate constitutes noise in the impedance respiration signal; and
removing the frequency component from the impedance respiration signal comprises, on a sample-by-sample basis:

determining a present estimate of the noise and a past estimate of the noise;
estimating the next estimate of the noise from the present estimate and the past estimate;
applying an adaptive filtering adjustment factor to the next estimate of the noise, the adaptive filtering adjustment factor having a scaled value of the error estimate limited to a maximum value of 1 to obtain an adjusted next estimate of the noise; and
subtracting the adjusted next estimate of the noise from the next sample of the impedance respiration signal.

6. The method of claim 5, wherein the adjusted next estimate of the noise is mathematically represented as:

$$e(nT + T) = e(nT + T) + \text{sign}(d) * \min\left(1, \frac{abs(d)}{10}\right)$$

where:

$n \equiv$ the sample number;
$T \equiv$ the sampling period; and
$d \equiv$ the error estimate.

7. The method of claim 5, wherein the adjusted next estimate of the noise is mathematically represented as:

$$e(n + 1) = 2 * \cos\left(2 * pi * \frac{f_{HR}}{f_s}\right) * e(n) - e(n - 1)$$

where:

$n \equiv$ the sample number;
$f_{HR} \equiv$ the patient's heart rate in Hz; and
$f_s \equiv$ the sampling rate of the respiration signal.

8.  The method of claim 7, wherein fHR = 0.25-5 Hz.

9.  The method of any one of claims 4 to 8, further comprising analyzing the filtered impedance respiration signal for patient conditions.

10. The method of claim 9, wherein:

    analyzing the filtered impedance respiration signal includes detecting an alarm condition; and
    the method further comprises issuing an alarm.

11. A physiological monitoring device, comprising:

    a processor-based resource; and
    a memory encoded with instructions that, when executed by the processor-based resource, performs any one of the methods of claims 1 to 10.

12. A system for physiologically monitoring a patient, the system comprising:

    a plurality of electrocardiogram ("ECG") sensors; and
    a physiological monitoring device communicating with the plurality of ECG sensors, the physiological monitor performing a method comprising:

        receiving an acquired impedance respiration signal associated with the patient's cardiac activity; and
        applying a non-linear, heart rate adaptive, cardiac artifact filter to obtain a filtered impedance respiration signal.

13. The system of claim 12, further comprising:

    a computing system communicating with the physiological monitoring device; and
    a records repository further comprising a plurality of electronic medical records ("ERMs") communicating with the physiological monitoring device through the computing system;
    wherein the physiological monitoring device:

        pushes information to at least one of the ERMs; and
        pulls information from at least one of the ERMs, which may be the same or different from the ERM to which the physiological monitoring device pushes information.

14. The system of claim 13, further comprising:

    a central monitoring station communicating with the physiological monitoring device through the computing system;
    wherein a caregiver monitors the patient's condition from the central monitoring station.

15. The system of claim 12, further comprising:

    a computing system communicating with the physiological monitoring device; and
    a central monitoring station communicating with the physiological monitoring device through the computing system;
    wherein a caregiver monitors the patient's condition from the central monitoring station.

FIG. 1

FIG. 2

FIG. 3

FIG. 4

500

510

ACQUIRE AN IMPEDANCE
RESPIRATION SIGNAL ASSOCIATED
WITH THE PATIENT'S
RESPIRATORY ACTIVITY

520

APPLY A NON-LINEAR, HEART RATE
ADAPTIVE, CARDIAC ARTIFACT
FILTER TO OBTAIN A FILTERED
IMPEDANCE RESPIRATION SIGNAL

*FIG. 5*

106

120

400a

400d

600

PHYSIOLOGICAL MONITORING DEVICE

PROCESSOR-BASED
RESOURCE

607

602

610

MEMORY

INSTRUCTIONS

615

DATA

605

*FIG. 6*

700

710

DETERMINE A VARYING HEART RATE
OF A MONITORED PATIENT FROM AN
ACQUIRED ELECTROCARDIOGRAM
("ECG") SIGNAL

720

ACQUIRE AN IMPEDANCE
RESPIRATION SIGNAL FROM THE
ACQUIRED ECG SIGNAL

730

APPLY A NON-LINEAR, HEART RATE
ADAPTIVE, CARDIAC ARTIFACT
FILTER TO OBTAIN A FILTERED
IMPEDANCE RESPIRATION SIGNAL

*FIG. 7*

FIG. 8

900

910

DETERMINE A PRESENT ESTIMATE
OF THE NOISE AND A PAST
ESTIMATE OF THE NOISE

920

ESTIMATE THE NEXT ESTIMATE OF
THE NOISE FROM THE PRESENT
ESTIMATE AND THE PAST ESTIMATE

930

APPLY AN ADAPTIVE FILTERING ADJUSTMENT FACTOR TO THE
NEXT ESTIMATE OF THE NOISE, THE ADAPTIVE FILTERING
ADJUSTMENT FACTOR HAVING A SCALED VALUE OF THE
ERROR ESTIMATE LIMITED TO A MAXIMUM VALUE OF 1 TO
OBTAIN AN ADJUSTED NEXT ESTIMATE OF THE NOISE

940

SUBTRACT THE ADJUSTED NEXT
ESTIMATE OF THE NOISE FROM THE
NEXT SAMPLE OF THE IMPEDANCE
RESPIRATION SIGNAL

FIG. 9

| | Europäisches Patentamt European Patent Office Office européen des brevets | **EUROPEAN SEARCH REPORT** | **Application Number** EP 24 21 3681 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/278711 A1 (MALLAS GEORGIOS [US]) 29 September 2016 (2016-09-29) * paragraph [0008] * * paragraph [0021] * * paragraph [0030] - paragraph [0036] * * paragraph [0053] * * figures 1, 2, 4, 6 * | 1-15 | INV. A61B5/00 A61B5/08 A61B5/053 A61B5/318 |
| A | US 2016/043704 A1 (LOU YAOLONG [SG] ET AL) 11 February 2016 (2016-02-11) * paragraph [0046] - paragraph [0057] * | 1-15 | |
| A | CRUTTENDEN COREY E ET AL: "Reference-free adaptive filtering of extracellular neural signals recording in ultra-high field magnetic resonance imaging scanners: Removal of periodic interferences", BIOMEDICAL SIGNAL PROCESSING AND CONTROL, ELSEVIER, AMSTERDAM, NL, [Online] vol. 71, 9 June 2021 (2021-06-09), XP086850273, ISSN: 1746-8094, DOI: 10.1016/J.BSPC.2021.102758 [retrieved on 2021-06-09] * page 6 * | 1-15 | |
| | | | TECHNICAL FIELDS SEARCHED (IPC) A61B |
| A | US 2014/232556 A1 (WILLIAMS STEVEN ALFRED [CN]) 21 August 2014 (2014-08-21) * figure 1 * | 1-15 | |

The present search report has been drawn up for all claims

| Place of search | Date of completion of the search | Examiner |
|---|---|---|
| The Hague | 14 January 2025 | Costa Angeli, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 4 559 382 A1

## ANNEX TO THE EUROPEAN SEARCH REPORT
## ON EUROPEAN PATENT APPLICATION NO.

EP 24 21 3681

This annex lists the patent family members relating to the patent documents cited in  the above-mentioned European search report.
The members are as contained in the European Patent Office EDP file on
The European Patent Office is in no way liable for these particulars which are merely given for the purpose of information.

14-01-2025

| Patent document cited in search report | Publication date | Patent family member(s) | Publication date |
|---|---|---|---|
| US 2016278711    A1 | 29-09-2016 | DE 102016105403 A1<br>US    2016278711 A1 | 29-09-2016<br>29-09-2016 |
| US 2016043704    A1 | 11-02-2016 | US    2014121548 A1<br>US    2016043704 A1<br>WO    2014070570 A1 | 01-05-2014<br>11-02-2016<br>08-05-2014 |
| US 2014232556    A1 | 21-08-2014 | NONE | |

For more details about this annex : see Official Journal of the European Patent Office, No. 12/82